# EUROPEAN PATENT APPLICATION

(11) **EP 4 631 949 A1**
(43) Date of publication of application: **15.10.2025**
(21) Application number: 23900240.5
(22) Date of filing: 21.08.2023
(51) Int. Cl.: C07D 519/00, A61K 9/10, A61K 31/4745, A61P 35/00

(54) **COMPOUND, NANOPARTICLES, MEDICINE AND METHOD FOR PRODUCING NANOPARTICLES**

(30) Priority: 09.12.2022 JP 2022197306
(71) Applicant: Japan Science and Technology Agency, Kawaguchi-shi, Saitama 332-0012 (JP)
(72) Inventor: KASAI Hitoshi, Sendai-shi, Miyagi 980-8577 (JP); KOSEKI Yoshitaka, Sendai-shi, Miyagi 980-8577 (JP)
(74) Representative: Gilani, Anwar
(86) International application number: PCT/JP2023/029978
(87) International publication number: WO 2024/122113

(57) **Abstract**

A compound represented by General Formula (1) or a salt thereof, in which each of R¹ and R² independently represents an alkyl group having 1 to 4 carbon atoms, R¹ and R² may be bonded to each other to form a ring together with a carbon atom to which R¹ and R² are bonded, and each R³ independently represents an alkylene group having 1 to 4 carbon atoms; nanoparticles containing the compound or a salt thereof; a medicine containing the compound or a salt thereof; and a method for producing nanoparticles, including a step of injecting a water-miscible organic solvent solution of the compound or a salt thereof into water are provided.

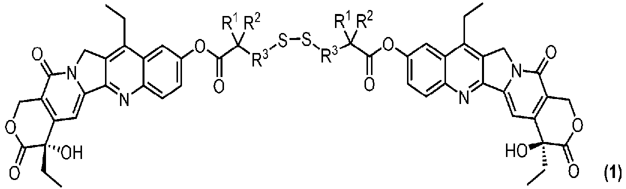

## Description

### TECHNICAL FIELD

The present invention relates to a compound, nanoparticle, a medicine, and a method for producing nanoparticles.

Priority is claimed on Japanese Patent Application No. 2022-197306, filed on December 9, 2022, the content of which is incorporated herein by reference.

### BACKGROUND ART

In recent years, in order to avoid various problems caused by a carrier, a carrier-free nano-drug has been developed. As a method for producing such a nano-drug, several methods such as a top-down method of crushing and fracturing drug plasma and a bottom-up method of producing nanoparticles from a molecule are known. Under such circumstances, the present inventors have already developed a unique method for producing organic nanoparticles which do not have a surfactant as a constitutional requirement, unlike micelles having a surfactant as a constitutional requirement (Patent Document 1). The present inventors have also reported that nanoparticles of an SN-38 derivative produced by the above-described method for producing nanoparticles have excellent anticancer activity (Patent Document 2).

### Citation List

### Patent Documents

Patent Document 1: Japanese Unexamined Patent Application, First Publication No. 2019-094260
Patent Document 2: PCT International Publication No. WO2022/190626

### SUMMARY OF INVENTION

### Technical Problem

An object of the present invention is to provide a novel compound or a salt thereof, capable of drug release in a tumor cell, nanoparticle containing the compound or a salt thereof, a medicine containing the compound or a salt thereof, and a method for producing the nanoparticles.

### Solution to Problem

The present invention includes the following aspects.
[1] A compound represented by General Formula (1) or a salt thereof. 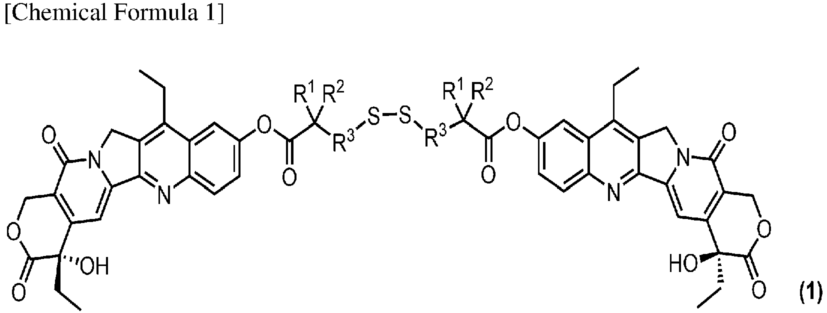 [in the formula, each of R¹ and R² independently represents an alkyl group having 1 to 4 carbon atoms, R¹ and R² may be bonded to each other to form a ring together with a carbon atom to which R¹ and R² are bonded, and each R³ independently represents an alkylene group having 1 to 4 carbon atoms]
[2] The compound or a salt thereof according to [1],
   in which ClogP is 5.0 to 14.0.
[3] The compound or a salt thereof according to [1] or [2],
   in which R¹ and R² in General Formula (1) are methyl groups.
[4] The compound or a salt thereof according to [1] or [2],
   in which R¹ and R² in General Formula (1) are ethyl groups.
[5] The compound or a salt thereof according to any one of [1] to [4],
   in which R³ in General Formula (1) is an ethylene group.
[6] The compound or a salt thereof according to any one of [1] to [4],
   in which R³ in General Formula (1) is a propylene group.
[7] Nanoparticles containing:
   the compound or a salt thereof according to any one of [1] to [6].
[8] A medicine containing:
   the compound or a salt thereof according to any one of [1] to [6].
[9] A medicine containing:
   the nanoparticle according to [7].
[10] The medicine according to [8],
   in which the medicine is used for prevention or treatment of cancer.
[11] The medicine according to [9],
   in which the medicine is used for prevention or treatment of cancer.
[12] A method for producing nanoparticles, including:
   a step of injecting a water-miscible organic solvent solution of the compound or a salt thereof according to any one of [1] to [6] into water.

### Advantageous Effects of Invention

According to the present invention, a novel compound or a salt thereof, capable of drug release in tumor cells, nanoparticle containing the compound or a salt thereof, a medicine containing the compound or a salt thereof, and a method for producing the nanoparticles are provided.

### BRIEF DESCRIPTION OF DRAWINGS

[FIG. 1] SEM images of nanoparticles of a compound (1-1) and nanoparticles of a compound (2-1) produced in Examples, in which the total width of the scales in the drawing is 1.0 µm, and the width of one scale is 0.1 µm.
[FIG. 2] Results of evaluating cytotoxic activity of compound (1-1), compound (2-1), and SN-38 against human breast cancer cell line MCF-7 in an in vitro activity test.
[FIG. 3] Results of evaluating release of SN-38 from compound (1-1) in PBS to which reduced dithiothreitol was added.
[FIG. 4] Results of evaluating release of SN-38 from compound (2-1) in PBS to which reduced dithiothreitol was added.
[FIG. 5] Results of evaluating release of SN-38 from compound (1-1) in PBS not containing reduced dithiothreitol.
[FIG. 6] Results of evaluating release of SN-38 from compound (2-1) in PBS not containing reduced dithiothreitol.
[FIG. 7] Results of evaluating release of SN-38 from compound (1-1) in PBS to which reduced glutathione was added.
[FIG. 8] Results of evaluating the release of SN-38 from compound (2-1) in PBS to which reduced glutathione was added.
[FIG. 9] Results of evaluating the release of SN-38 from a compound (2-2) in PBS to which reduced glutathione was added.
[FIG. 10] Results of evaluating the release of SN-38 from a compound (2-3) in PBS to which reduced glutathione was added.

### DESCRIPTION OF EMBODIMENTS

### [Compound or salt thereof]

A first aspect according to the present invention is a compound represented by General Formula (1) (hereinafter, also referred to as "compound (1)") or a salt thereof. 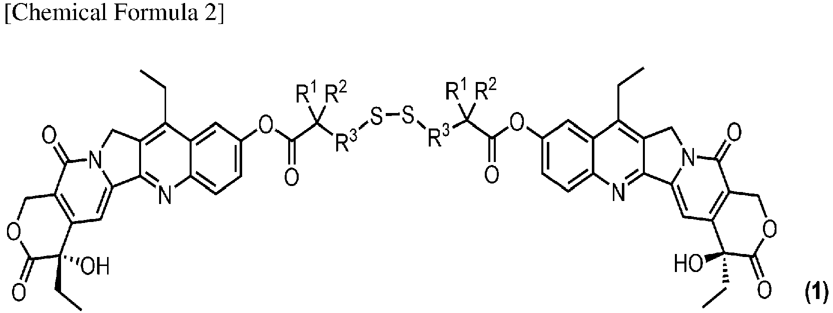 [in the formula, each of R¹ and R² independently represents an alkyl group having 1 to 4 carbon atoms, R¹ and R² may be bonded to each other to form a ring together with a carbon atom to which R¹ and R² are bonded, and each R³ independently represents an alkylene group having 1 to 4 carbon atoms]

In Formula (1), R¹ and R² may be a linear alkyl group or a branched alkyl group, but is preferably a linear alkyl group. The number of carbon atoms in the linear alkyl group of R¹ and R² is preferably 1 to 3, more preferably 1 or 2, and still more preferably 1. Examples of the linear alkyl group include a methyl group, an ethyl group, an n-propyl group, and an n-butyl group. The number of carbon atoms in the branched alkyl group of R¹ and R² is preferably 3 or 4, and more preferably 3. Examples of the branched alkyl group include an isopropyl group, an isobutyl group, a tert-butyl group, and a sec-butyl group.

R¹ and R² may be the same or different from each other. From the viewpoint of ease of synthesis, it is preferable that R¹ and R² be the same. Two R¹'s may be the same or different from each other. From the viewpoint of ease of synthesis, it is preferable that two R¹'s be the same. Two R²'s may be the same or different from each other. From the viewpoint of ease of synthesis, it is preferable that two R²'s be the same.

Examples of a preferred combination of R¹ and R² include a combination in which all R¹ and R² are methyl groups; a combination in which all R¹ and R² are ethyl groups; a combination in which all R¹ and R² are n-propyl groups; a combination in which all R¹ and R² are n-butyl groups; a combination in which all R¹ and R² are isopropyl groups; a combination in which all R¹ and R² are isobutyl groups; a combination in which all R¹ and R² are tert-butyl groups; a combination in which all R¹ and R² are sec-butyl groups; a combination in which R¹ is a methyl group and R² is an ethyl group; a combination in which R¹ is a methyl group and R² is an n-propyl group; a combination in which R¹ is a methyl group and R² is an n-butyl group; a combination in which R¹ is an ethyl group and R² is an n-propyl group; a combination in which R¹ is an ethyl group and R² is an n-butyl group; a combination in which R¹ is an n-propyl group and R² is an n-butyl group, and the like. Among the above, it is preferable that R¹ and R² be a methyl group or an ethyl group, it is more preferable that all R¹ and R² be methyl groups or all R¹ and R² are ethyl groups, and it is still more preferable that all R¹ and R² be methyl groups.

R¹ and R² may be bonded to each other to form a ring with a carbon atom to which R¹ and R² are bonded. Preferred examples of a group formed by bonding R¹ and R² to each other include an alkylene group having 4 or 5 carbon atoms. More specific examples of the cyclic group formed by R¹ and R² with the carbon atom to which R¹ and R² are bonded include a cyclobutyl group and a cyclohexyl group.

In Formula (1), R³ may be a linear alkylene group or a branched alkylene group, but is preferably a linear alkylene group. The number of carbon atoms in the linear alkylene group of R³ is preferably 2 or 3, and more preferably 2. Examples of the linear alkylene group of R³ include a methylene group, an ethylene group, an n-propylene group, and an n-butylene group. The number of carbon atoms in the branched alkylene group of R³ is preferably 2 to 4, and more preferably 3. Examples of the branched alkylene group of R³ include an alkylmethylene group such as -CH(CH₃)-, - CH(CH₂CH₃)-, -C(CH₃)₂-, and -C(CH₃)(CH₂CH₃)-; an alkylethylene group such as - CH(CH₃)CH₂-, -CH(CH₃)CH(CH₃)-, -C(CH₃)₂CH₂-, and -CH(CH₂CH₃)CH₂-; and an alkyltrimethylene group such as -CH(CH₃)CH₂CH₂- and -CH₂CH(CH₃)CH₂-.

Two R³'s may be the same or different from each other. From the viewpoint of ease of synthesis, it is preferable that two R³'s be the same. It is preferable that R³ be an ethylene group or an n-propylene group, it is more preferable that both of two R³' s be ethylene groups or n-propylene groups, and it is still more preferable that both of two R³'s be ethylene groups.

Specific examples of compound (1) are shown below, but the present invention is not limited thereto.

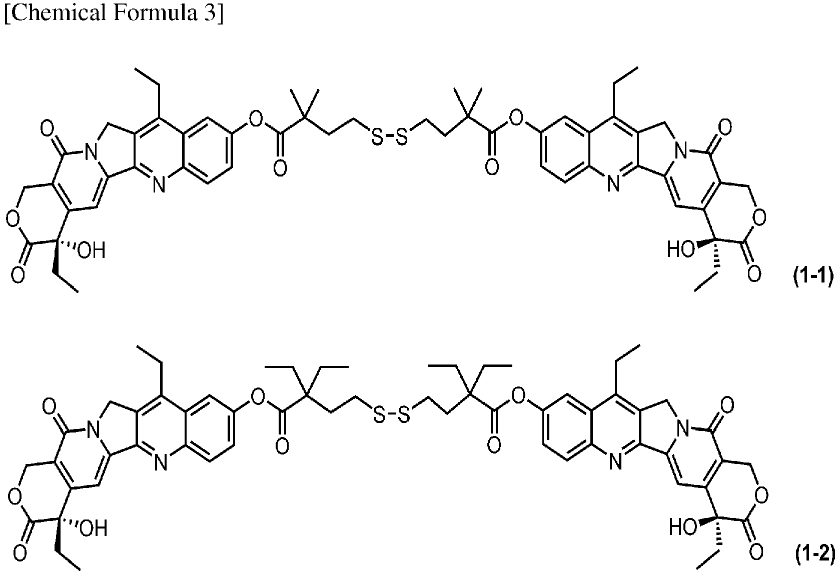

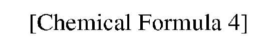

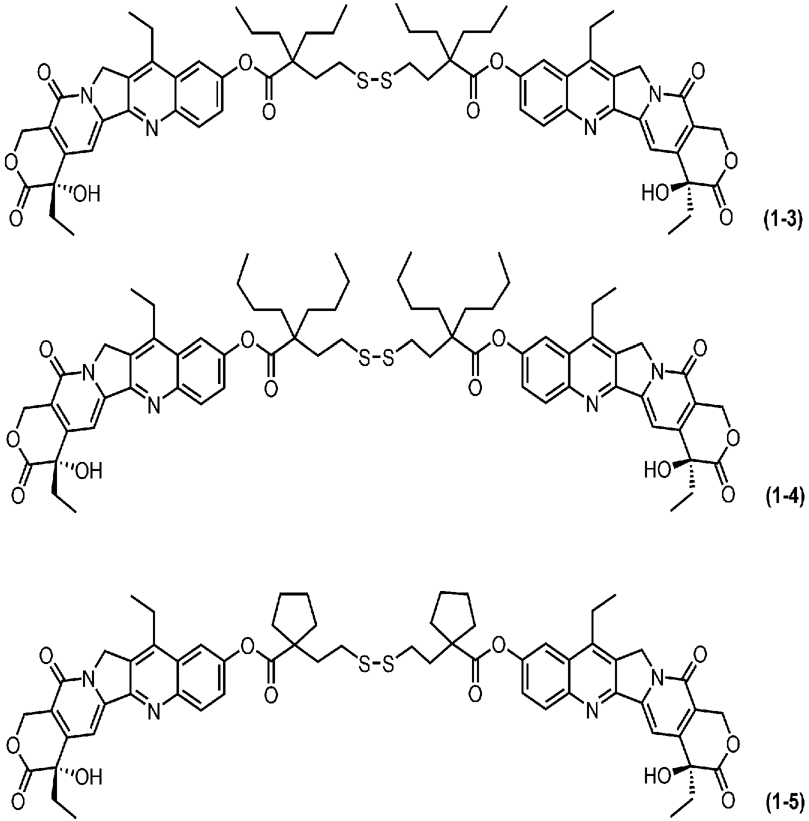

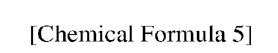

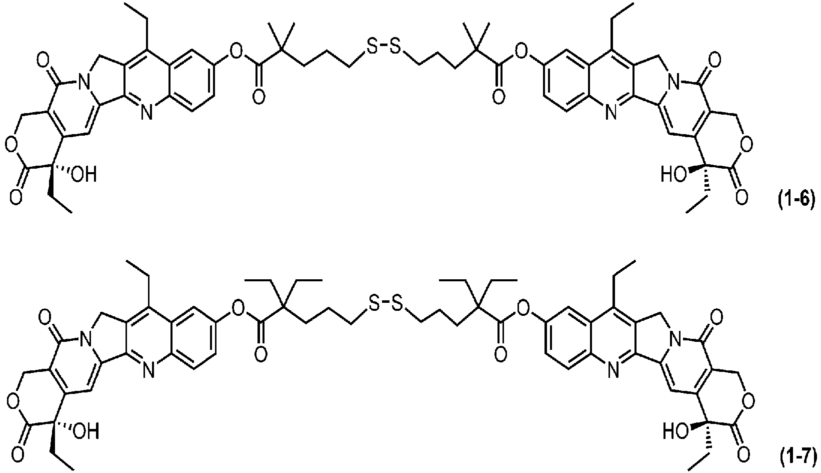

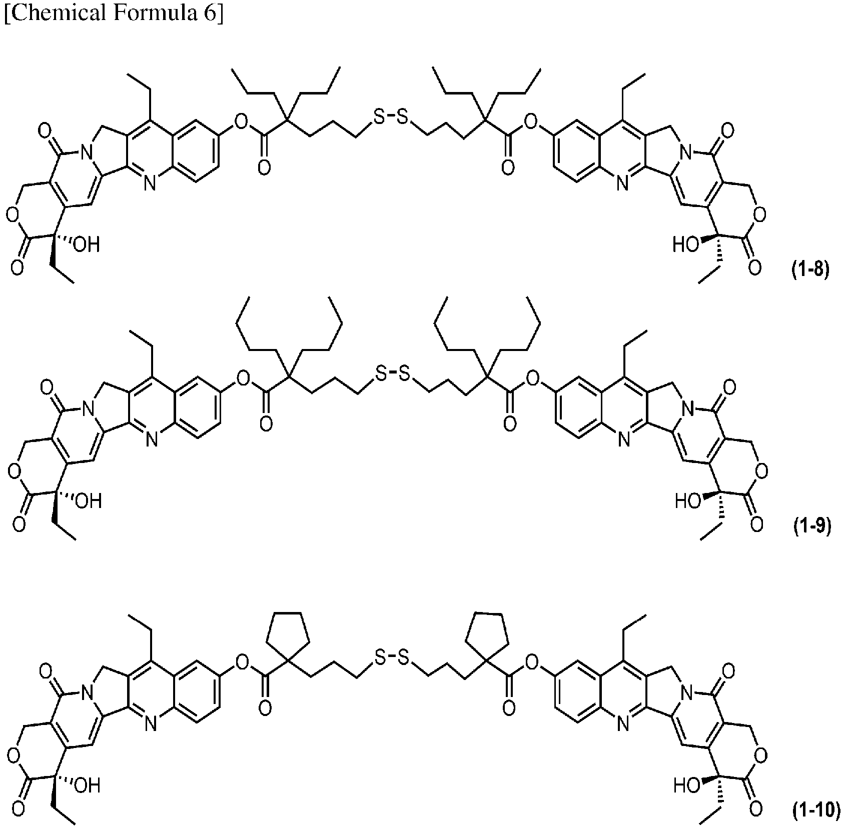

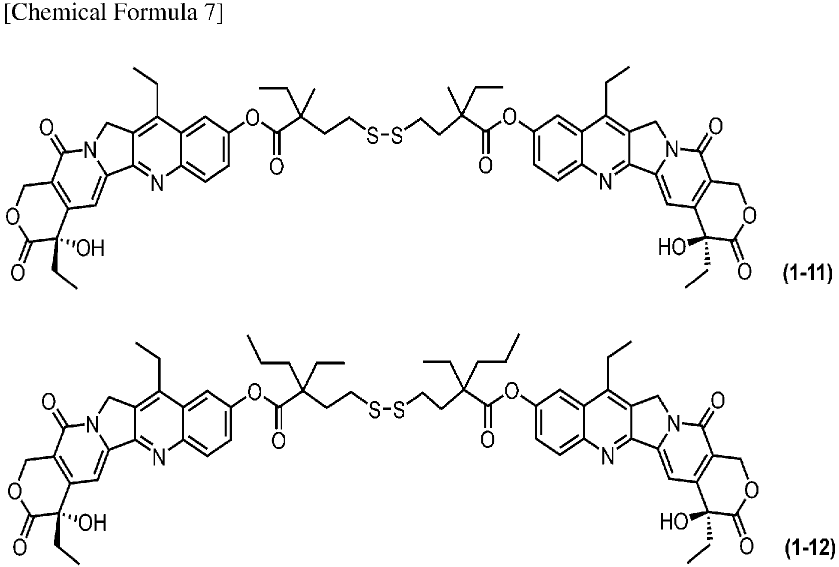

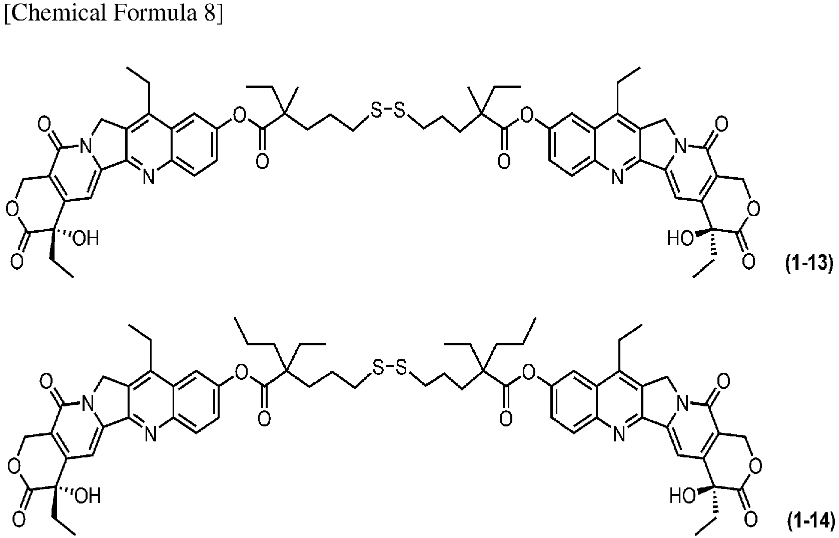

Compound (1) may be in the form of a salt. The term "salt" means a pharmaceutically acceptable salt. The salt of compound (1) includes an acid addition salt and a salt with a base. Specific examples of the acid addition salt include an inorganic acid salt such as hydrochloride, hydrobromide, hydroiodide, sulfate, perchlorate, and phosphate; an organic acid salt such as oxalate, malonate, succinate, maleate, fumarate, lactate, malate, citrate, tartrate, benzoate, trifluoroacetate, acetate, methanesulfonate, p-toluenesulfonate, and trifluoromethanesulfonate; and an acidic amino acid salt such as glutamate and aspartate. Specific examples of the salt with a base include an alkali metal salt such as a sodium salt and a potassium salt; an alkaline earth metal salt such as a calcium salt and a magnesium salt; a salt with an organic base such as a pyridine salt and a triethylamine salt; and a salt with a basic amino acid such as lysine and arginine.

Compound (1) or a salt thereof may be in the form of a hydrate or a solvate. Examples of a solvent forming the solvate include alcohols such as ethanol and propanol; organic acids such as acetic acid; esters such as ethyl acetate; ethers such as tetrahydrofuran and diethyl ether; ketones such as acetone; and dimethyl sulfoxide (DMSO).

When an isomer such as a geometrical isomer, a stereoisomer, and an optical isomer is present as compound (1), the isomer is included in compound (1) unless otherwise specified.

Compound (1) or a salt thereof is used as an antitumor agent, more specifically, as a prodrug of an antitumor agent. Therefore, it is preferable that compound (1) or a salt thereof suppress hydrolysis in blood (release of an active ingredient from a prodrug molecule due to cleavage of a carbonate moiety), and generate SN-38 or a derivative thereof as the active ingredient by hydrolysis in tumor cells.

As shown in Examples described later, compound (1) is stable in the absence of a reducing agent (reduced dithiothreitol (DTT) or reduced glutathione (GSH)), and thus the release of the active ingredient (SN-38 or a derivative thereof) does not occur. On the other hand, compound (1) releases the active ingredient (SN-38 or a derivative thereof) in the presence of the reducing agent (reduced DTT or reduced GSH).

GSH plays an important role in the metabolism of the living body as an in vivo reducing agent having a low molecular weight. In general, the GSH concentration in tumor cells is higher than that in normal cells. It has been reported that the GSH concentration in tumor cells is 100 to 1,000 times that outside cells. The main reason for this is that tumor cells acquire a mechanism to protect themselves from oxidative stress, which is necessary for the proliferation of tumor cells (Traverso, N. et al., Oxid Med Cell Longev. 2013; 2013: 972913; Research report of scientific research funding project, June 3, 2017, project number: 15H06175, research subject name (Japanese): Elucidation of oxidative stress resistance mechanism in head and neck cancer using glutathione-sensitive fluorescent probe, research representative: Masashi Yoshida). GSH in cells is usually present in an almost entirely reduced state (98% or more).

Tumor cells acquire a metabolic pathway different from that of normal cells in order to generate energy necessary for proliferation and survival. In normal cells, energy is usually generated by oxidative phosphorylation, which uses oxygen to generate ATP. On the other hand, in tumor cells, energy is generated by aerobic glycolysis (Warburg effect). In the aerobic glycolysis pathway, glucose is metabolized to pyruvate, and then pyruvate is metabolized to lactic acid. In tumor cells, reduced glutathione is synthesized by the pentose phosphate pathway, which is an aerobic glycolysis side pathway, and it is said that the intracellular concentration of reduced glutathione is increased. It is said that tumor cells have a higher reduced glutathione concentration than that in normal cells, which results in oxidative stress resistance.

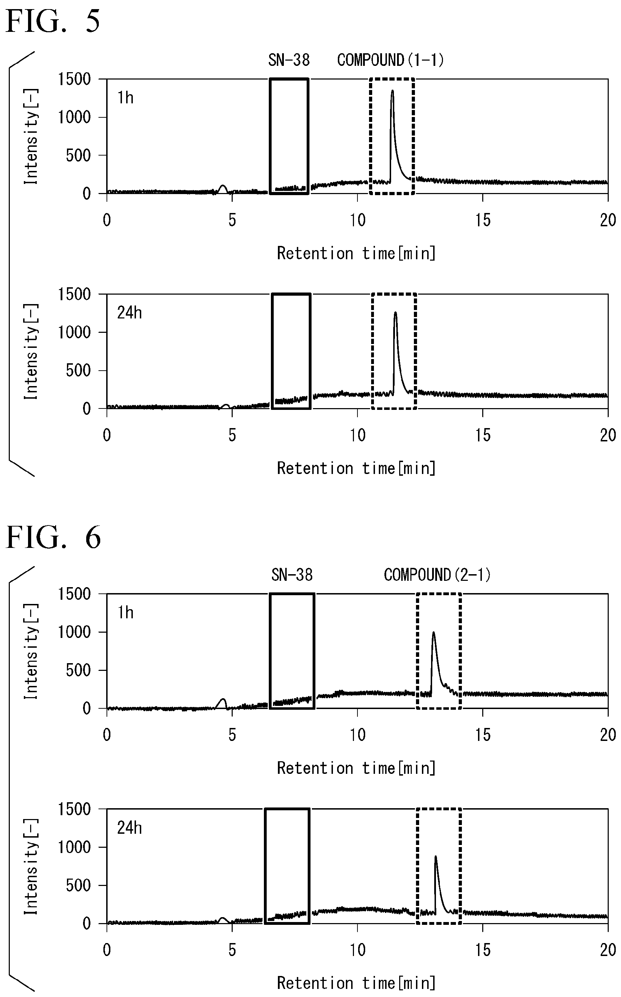

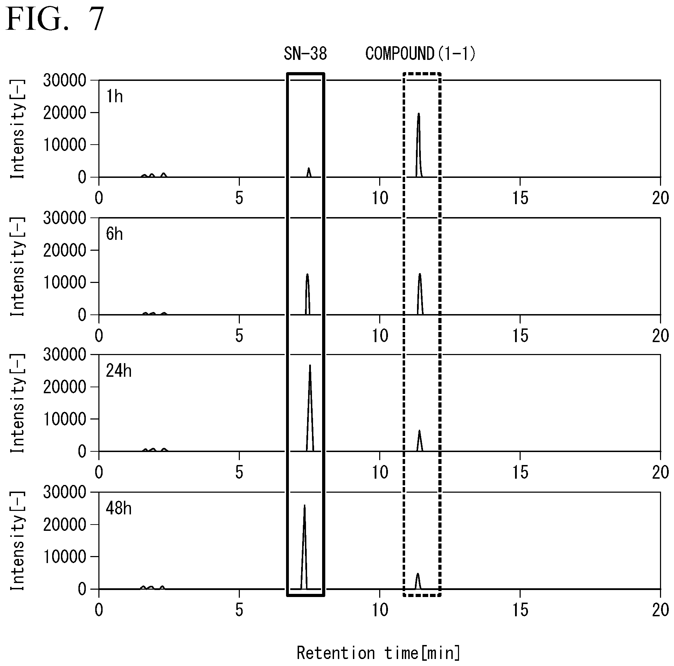

As shown in Examples described later, compound (1) releases SN-38 or a derivative thereof as an active ingredient in the presence of reduced glutathione (see FIG. 7). In addition, compound (1) is stably maintained in normal cells without releasing SN-38 (see FIG. 6).

Therefore, compound (1) can be suitably used as a prodrug-type anticancer agent which specifically releases an active ingredient in tumor cells. Nanoparticles containing compound (1) continuously release SN-38 or a derivative thereof in the presence of a reducing agent such as reduced glutathione. Therefore, a drug containing nanoparticles containing compound (1) can be used as a sustained-release agent of SN-38 or a derivative thereof.

From the viewpoint of hydrolysis resistance in blood, dispersion stability required in production of nanoparticles, selective drug release properties in cancer cells, and the like, compound (1) preferably has a ClogP of 5.0 to 14.0 and more preferably has a ClogP of 5.0 to 12.0. ClogP is LogP, which is an octanol/water partition coefficient calculated from the structural formula of a target compound using the "ClogP" ChemDraw Professional 16.0 program.

Table 1 shows ClogP values of representative compounds of compound (1) (compounds (1-1) to (1-14)).

**[Table 1]**

| Compound | ClogP |
|---|---|
| Compound (1-1) | 5.9 |
| Compound (1-2) | 8.0 |
| Compound (1-3) | 10.1 |
| Compound (1-4) | 11.9 |
| Compound (1-5) | 7.4 |
| Compound (1-6) | 6.5 |
| Compound (1-7) | 8.7 |
| Compound (1-8) | 10.8 |
| Compound (1-9) | 12.5 |
| Compound (1-10) | 8.1 |
| Compound (1-11) | 7.0 |
| Compound (1-12) | 9.1 |
| Compound (1-13) | 7.6 |
| Compound (1-14) | 9.7 |

### <Method for producing compound (1)>

Compound (1) can be produced by various methods. Compound (1) can be produced, for example, by the reaction of Formula (I) (hereinafter, also referred to as "reaction (I)"). 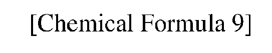 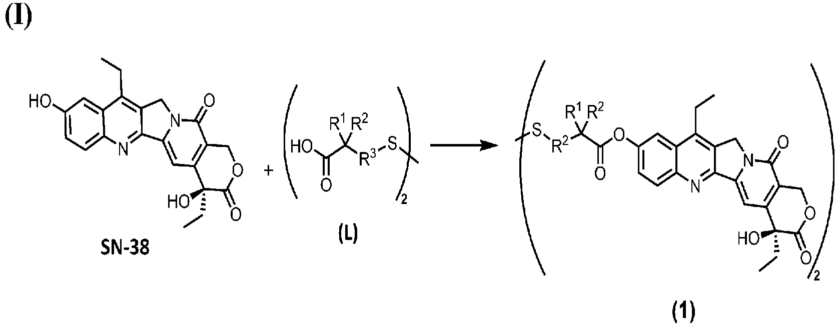 [in the formula, each of R¹ and R² independently represents an alkyl group having 1 to 4 carbon atoms, R¹ and R² may be bonded to each other to form a ring together with a carbon atom to which R¹ and R² are bonded, and each R³ independently represents an alkylene group having 1 to 4 carbon atoms]

In reaction (I), compound (1) is obtained by reacting SN-38 with a compound (L). The ratio of amount of SN-38 and the above-described compound (L) used is not particularly limited, but for example, compound (L) can be used in an amount of 0.4 to 0.5 mol with respect to 1 mol of the SN-38. The reaction temperature of reaction (I) is not particularly limited, and is usually carried out at room temperature, under cooling, or under heating conditions. The reaction temperature may be, for example, 0 to 90°C, and is preferably 20 to 90°C. The reaction time of reaction (I) is not particularly limited, but can be set to, for example, 1 to 50 hours.

Reaction (I) can be carried out in the presence of a base such as 4-dimethylaminopyridine (DMAP), triethylamine (TEA), and pyridine. In reaction (I), a carbodiimide-based condensing agent such as 1-ethyl-3-(3-dimethylaminopropyl)carbodiimide hydrochloride (EDC·HCl) may be used.

Reaction (I) can usually be carried out in a conventional solvent that does not adversely affect the reaction. Examples of the reaction solvent include dichloromethane, triethylamine, acetone, methyl ethyl ketone, tetrahydrofuran, dioxane, diethyl ether diglyme, acetonitrile, N,N-dimethylformamide, dimethyl sulfoxide, and a mixed solvent of two or more selected from these solvents.

Compound (L) used in reaction (I) can be produced by various methods. Compound (L) can be produced, for example, by the reaction of Formula (II) (hereinafter, also referred to as "reaction (II)") and the reaction of Formula (III) (hereinafter, also referred to as "reaction (111)"). 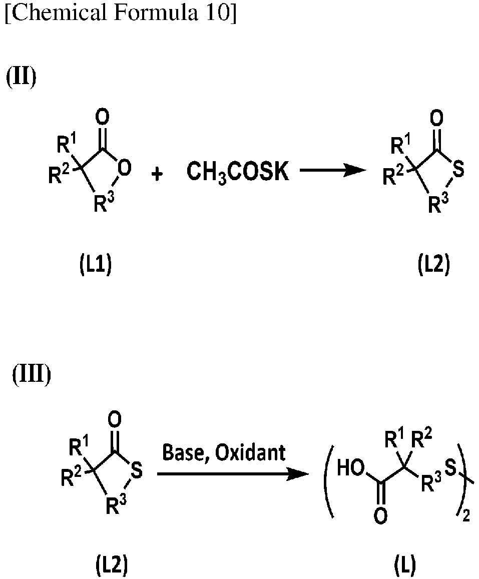 [in the formula, each of R¹ and R² independently represents an alkyl group having 1 to 4 carbon atoms, R¹ and R² may be bonded to each other to form a ring together with a carbon atom to which R¹ and R² are bonded, and each R³ independently represents an alkylene group having 1 to 4 carbon atoms]

In reaction (II), a compound (L1) and potassium thioacetate (CH₃COSK) are reacted with each other to obtain a compound (L2). The ratio of amount of compound (L1) and potassium thioacetate used is not particularly limited, but for example, the latter can be used in an amount of 1 mol or more with respect to 1 mol of the former. It is preferable that the latter be used in an amount of 1.2 to 1.6 mol with respect to 1 mol of the former. The reaction temperature of reaction (II) is not particularly limited, and is usually carried out under heating conditions. The reaction temperature may be, for example, 100 to 200°C, and is preferably 120 to 170°C. The reaction time of reaction (II) is not particularly limited, but can be set to, for example, 1 to 24 hours.

(Compound (L1) may be a commercially available product, or may be prepared based on a known document or the like describing a synthesis method of the compound.

Reaction (II) can usually be carried out in a conventional solvent that does not adversely affect the reaction. Examples of the reaction solvent include a high-boiling polar solvent such as dimethylacetamide (DMA).

In reaction (III), compound (L2) is hydrolyzed in the presence of a base, and then a disulfide bond is formed in the presence of an oxidizing agent to obtain compound (L). Examples of the base include potassium hydroxide and sodium hydroxide. Examples of the oxidizing agent include iodine. Iodine can be dissolved in potassium iodide and then added to the reaction solution. The reaction temperature of reaction (III) is not particularly limited, and is usually carried out at room temperature, under cooling or heating conditions. The reaction temperature of the hydrolysis reaction may be, for example, 0 to 120°C, and is preferably 20 to 110°C. The reaction temperature of the oxidation reaction may be, for example, 0 to 50°C, and is preferably 20 to 50°C. The reaction time of reaction (III) is not particularly limited. For example, the hydrolysis reaction can be carried out for 1 to 24 hours. For example, the oxidation reaction can be carried out for 1 to 24 hours.

Reaction (III) can usually be carried out in a conventional solvent that does not adversely affect the reaction. Examples of the reaction solvent include water, dichloromethane, triethylamine, acetone, methyl ethyl ketone, tetrahydrofuran, dioxane, diethyl ether diglyme, acetonitrile, N,N-dimethylformamide, dimethyl sulfoxide, and a mixed solvent of two or more selected from these solvents.

### [Nanoparticles]

A second aspect according to the present invention is nanoparticles containing compound (1) or a salt thereof. The term "nanoparticles" refers to particles having an average particle diameter of less than 1 µm. In a typical embodiment, the nanoparticles have a substantially spherical shape. The average particle diameter of the nanoparticles can be measured by a scanning electron microscope (SEM) or a dynamic light-scattering method (DLS). The average particle diameter of the nanoparticles is preferably 10 to 500 nm, and more preferably 10 to 100 nm. Typically, the nanoparticles can be prepared by injecting a water-miscible organic solvent solution of compound (1) or a salt thereof into water and dispersing the solution by a method described later. Therefore, in a typical embodiment, the nanoparticles according to the present aspect are organic nanocrystals of compound (1) or a salt thereof, and thus consist substantially of only compound (1) or a salt thereof. However, the nanoparticles according to the present aspect may contain a component other than compound (1) or a salt thereof (for example, an additive to a water-miscible organic solvent used for producing the nanoparticles), as long as the effect of the present invention is obtained. The content of compound (1) or a salt thereof in the nanoparticles is, for example, preferably 95% by mass or more, more preferably 99% by mass or more, and still more preferably 99.9% by mass or more.

### [Method for producing nanoparticles]

A third aspect according to the present invention is a method for producing the nanoparticles according to the second aspect. The production method according to the present aspect includes a step of injecting a water-miscible organic solvent solution of compound (1) or a salt thereof into water (hereinafter, referred to as "injection step").

### <Injection step>

A method of injecting a water-miscible organic solvent solution of compound (1) or a salt thereof (hereinafter, also simply referred to as "water-miscible organic solvent solution") into water is not particularly limited. For example, the water-miscible organic solvent solution can be injected into water using a syringe or the like. Compound (1) or a salt thereof is crystallized or granulated by injecting the water-miscible organic solvent solution into water. As a result, an aqueous dispersion liquid of nanoparticles of compound (1) or a salt thereof can be obtained.

The term "water-miscible organic solvent" refers to an organic solvent having miscibility with water. The water-miscible organic solvent is not particularly limited as long as it is a suitable solvent for compound (1) or a salt thereof. Examples of the water-miscible organic solvent include tetrahydrofuran, acetone, dioxane, acetonitrile, methanol, ethanol, propanol, N-methylpyrrolidone, and dimethyl sulfoxide. From the viewpoint of solubility and safety of compound (1), the water-miscible organic solvent is preferably acetone, tetrahydrofuran, ethanol, dimethyl sulfoxide, or the like. These solvents can be used alone or in combination of two or more kinds thereof.

The content of compound (1) or a salt thereof in the water-miscible organic solvent solution is not particularly limited, but can be appropriately set, for example, in the range of 0.1 to 15% by mass, preferably in the range of 0.1 to 10% by mass. In addition to compound (1) or a salt thereof, polysorbate 80 (PS80), polyvinylpyrrolidone (PVP), or the like may be added to the water-miscible organic solvent solution. An amount of the water-miscible organic solvent solution of compound (1) or a salt thereof to be injected into water is not particularly limited, but for example, 0.1 to 1 mL, preferably 0.1 to 0.2 mL of the water-miscible organic solvent solution can be added to 10 mL of water. An injection time is not particularly limited, but for example, it is preferably 0.1 to 1 second and more preferably 0.1 to 0.2 seconds. The temperature in a case of carrying out the injection step is not particularly limited, but can be appropriately set, for example, in the range of 0 to 30°C, preferably in the range of 10 to 20°C.

In the dispersion liquid of the nanoparticles obtained by the production method according to the present aspect, a concentration of compound (1) or a salt thereof is, for example, 0.01 to 100 mM, preferably 0.05 to 10 mM.

### <Other steps>

The production method according to the present aspect may include other steps in addition to the above-described injection step. Examples of the other steps include a stirring step, a water-miscible organic solvent removing step, and a nanoparticle isolating step.

### Stirring step:

The production method according to the present aspect may include a stirring step after the above-described injection step. The stirring step is a step of stirring the solution after injecting the water-miscible organic solvent solution of compound (1) or a salt thereof into water. By carrying out the stirring step, crystallization or granulation of compound (1) or a salt thereof can be promoted. The stirring speed is not particularly limited, but can be appropriately set, for example, in the range of 1,000 to 1,500 rpm, preferably in the range of 1,200 to 1,500 rpm. The temperature in the stirring step may be the same as that in the injection step. The stirring time is not particularly limited, but can be appropriately set, for example, in the range of 1 to 10 seconds, preferably in the range of 1 to 3 seconds.

### Water-miscible organic solvent removing step:

The dispersion liquid of the nanoparticles of compound (1) or a salt thereof obtained by performing the above-described injection step and optionally the stirring step contains a water-miscible organic solvent. When the dispersion liquid of the nanoparticles is used as it is, the water-miscible organic solvent may be removed before use, from the viewpoint of safety and the like. Therefore, the production method according to the present aspect may include the water-miscible organic solvent removing step. A method of removing the water-miscible organic solvent is not particularly limited, and a known method can be used. Examples of the method of removing the water-miscible organic solvent include distillation under reduced pressure (or under normal pressure) and dialysis.

### Nanoparticle isolating step:

The production method according to the present aspect may include a step of isolating the nanoparticles from the dispersion liquid of the nanoparticles. For example, the nanoparticles can be isolated as fine particle powder by performing a solid-liquid separation operation such as filtration on the dispersion liquid of the nanoparticles.

In the production method according to the present aspect, for example, the above-described steps can be appropriately combined and carried out. The production method according to the present aspect can be carried out using compound (1) or a salt thereof as a raw material, with reference to the description in Japanese Unexamined Patent Application No. 2019-94260.

### [Medicine]

A fourth aspect according to the present invention is a medicine containing compound (1) or a salt thereof.

In the medicine according to the present aspect, compound (1) or a salt thereof may be used alone as the medicine. Alternatively, the above-described nanoparticles containing compound (1) or a salt thereof may be used as the medicine.

The medicine according to the present aspect may be a pharmaceutical composition containing compound (1) or a salt thereof and a pharmaceutically acceptable carrier. In one embodiment, compound (1) or a salt thereof may be contained in the pharmaceutical composition as the above-described nanoparticles. The above-described pharmaceutical composition contains compound (1) or a salt thereof as an active ingredient. The term "pharmaceutically acceptable carrier" means a carrier that does not inhibit physiological activity of an active ingredient and does not exhibit substantial toxicity to a target of administration of the active ingredient. The phrase "does not exhibit substantial toxicity" means that, at the dosage in which a component is usually used, the component does not exhibit toxicity in the target of administration. Typically, the pharmaceutically acceptable carrier includes various known pharmaceutically acceptable components regarded as non-active ingredients. Specific examples of pharmaceutically acceptable carriers include an isotonic agent, a chelating agent, a stabilizer, a pH adjuster, a preservative, an antioxidant, a dissolution aid, and a thickener.

Examples of the isotonic agent include sugars such as glucose, trehalose, lactose, fructose, mannitol, xylitol, and sorbitol; polyhydric alcohols such as glycerin, polyethylene glycol, and propylene glycol; and inorganic salts such as sodium chloride, potassium chloride, and calcium chloride.

Examples of the chelating agent include edetates such as disodium edetate, calcium disodium edetate, trisodium edetate, tetrasodium edetate, and calcium edetate; ethylenediaminetetraacetate; nitrilotriacetate or a salt thereof; sodium hexametaphosphate; and citric acid.

Examples of the stabilizer include sodium hydrogen sulfite.

Examples of the pH adjuster include acids such as hydrochloric acid, carbonic acid, acetic acid, and citric acid; alkali metal hydroxides such as sodium hydroxide and potassium hydroxide; alkali metal carbonates or hydrogen carbonates such as sodium carbonate; alkali metal acetates such as sodium acetate; alkali metal citrates such as sodium citrate; and bases such as trometamol.

Examples of the preservative include sorbic acid, potassium sorbate; parahydroxybenzoate such as methyl 4-hydroxybenzoate, ethyl 4-hydroxybenzoate, propyl 4-hydroxybenzoate, and butyl 4-hydroxybenzoate; chlorhexidine gluconate; quaternary ammonium salts such as benzalkonium chloride, benzethonium chloride, and cetylpyridinium chloride; alkyl polyamino ethyl glycine; chlorobutanol; polyquaternium; polyhexamethylene biguanide; and chlorhexidine.

Examples of the antioxidant include sodium hydrogensulfite, dried sodium sulfite, sodium pyrosulfite, and concentrated mixed tocopherol.

Examples of the dissolution aid include sodium benzoate, glycerin, D-sorbitol, glucose, propylene glycol, hydroxypropyl methylcellulose, polyvinylpyrrolidone, macrogol, and D-mannitol.

Examples of the thickener include polyethylene glycol, methylcellulose, ethylcellulose, carmellose sodium, xanthan gum, sodium chondroitin sulfate, hydroxyethylcellulose, hydroxypropylcellulose, hydroxypropyl methylcellulose, polyvinylpyrrolidone, and polyvinyl alcohol.

The pharmaceutical composition may further contain a compound known to have an antitumor effect, in addition to compound (1) or a salt thereof. Examples of the compound known to have an antitumor effect include a chemotherapeutic agent such as podophyllotoxin (PPT), doxorubicin (DOX), and fluorouracil (5-FU); but the present invention is not limited thereto.

The content of compound (1) or a salt thereof in the pharmaceutical composition is not particularly limited, and can be appropriately set to, for example, 90% by mass or more, 70% by mass or more, 50% by mass or more, 30% by mass or more, 10% by mass or more, 5% by mass or more, or 1% by mass or more in terms of the content of compound (1). The pharmaceutical composition may contain compound (1) or a salt thereof in a therapeutically effective amount. The term "therapeutically effective amount" means the amount of medication effective for the treatment or prevention of a target disease. For example, the therapeutically effective amount of compound (1) or a salt thereof may be an amount effective for treatment or prevention of a tumor. The therapeutically effective amount may be appropriately determined according to symptoms, body weight, age, gender, and the like of a patient, the dosage form of the pharmaceutical composition, the administration method, and the like.

The dosage form of the pharmaceutical composition is not particularly limited, and examples thereof include various dosage forms, for example, oral administration agents such as tablets, pills, capsules, powders, granules, syrups, and sublingual agents; parenteral administration agents such as injection agents (intravenous injection, intramuscular injection, local injection, and the like), gargles, drip infusion agents, external preparations (ointments, creams, patches, inhalants), and suppositories. Among the above-described dosage forms, preferred examples thereof include an oral agent, an injection agent (intravenous injection), and a drip infusion agent. In one embodiment, compound (1) or a salt thereof is also useful since it exhibits an antitumor effect even in a method of administering compound (1) or a salt thereof by direct injection into a tumor.

The medicine according to the present aspect can be used for prevention or treatment of cancer. The cancer to be prevented or treated is not particularly limited, but examples thereof preferably include a solid tumor. Specific examples thereof include cancers occurring in hollow organs (so-called viscera), such as esophageal cancer, gastric cancer, colon cancer, large intestinal cancer, rectal cancer, pancreatic cancer, liver cancer, laryngeal cancer, lung cancer, prostate cancer, bladder cancer, breast cancer, uterine cancer, and ovarian cancer. Examples of a target site include tumor cells, tumor tissue, and organs or viscera in which tumor tissue is present, and the inside thereof.

In addition, from the viewpoint of physical properties and pharmacological action of the medicine according to the present aspect, as general drug administration (for example, oral administration, intravenous administration, percutaneous administration, local administration, and the like), the target organ is preferably an intraluminal organ (so-called, a visceral organ) such as a digestive organ, a circulatory organ, a respiratory organ, a urinary organ, and a reproductive organ; and a sensory organ (eye, ear, nose, skin, and the like), a motor organ (bone, muscle, joint, and the like), and the like, in view of absorption in the small intestine, the existence of the blood-brain barrier, and the like.

A dose of the medicine according to the present aspect varies depending on the administration route, the age, weight, and symptoms of the patient, and cannot be generally defined; and examples thereof include a daily dose of compound (1) or a salt thereof of approximately 10 to 5,000 mg, and preferred examples thereof include a daily dose of compound (1) or a salt thereof of approximately 100 to 1,000 mg. When the medicine according to the present aspect is administered once a day, it is sufficient that the medicine be provided in the above amount in one preparation; and when the medicine according to the present aspect is administered three times a day, it is sufficient that the medicine be provided in 1/3 of the above amount in one preparation.

The medicine according to the present aspect is administered to a patient or an affected animal such as a mammal. Examples of mammals include humans, monkeys, mice, rats, rabbits, cats, dogs, pigs, cows, horses, and sheep; but the mammals are not limited thereto.

Since the medicine according to the present aspect contains the nanoparticles containing compound (1) or a salt thereof, the medicine continuously releases SN-38 under reducing conditions such as in the presence of reduced GSH. It is expected that the release of SN-38 from the nanoparticles containing compound (1) occurs relatively gradually, and thus it is possible to suppress the risk of SN-38 being rapidly released in excess. Therefore, even when the dose of compound (1) in one administration is increased, the risk of causing side effects can be avoided. In addition, it is also expected that the number of administrations can be reduced and the administration interval can be lengthened. SN-38 is a DNA topoisomerase I inhibitor having an antitumor effect. Therefore, the medicine according to the present aspect is expected to be used as a sustained-release anticancer agent.

### [Other embodiments]

In one aspect, the present invention provides a method for treating or preventing cancer, which includes administering compound (1) or a salt thereof, or the above-described nanoparticles to a subject. Examples of the dose include a therapeutically effective amount, and specific examples thereof include the above-described dose.

In one aspect, the present invention provides compound (1) or a salt thereof, or the above-described nanoparticles for the treatment or prevention of cancer.

In one aspect, the present invention provides the use of compound (1) or a salt thereof, or the above-described nanoparticles for producing a pharmaceutical composition for the treatment or prevention of cancer.

### Examples

Hereinafter, the present invention will be described with reference to Examples, but the present invention is not limited to Examples.

### [Synthesis Example 1]

A compound (A1) (2.00 g, 17.5 mmol) and potassium thioacetate (CH₃COSK) (3.14 g, 27.5 mmol) were dissolved in dimethylacetamide (DMA) (12.6 mL). The obtained solution was reacted at 150°C for 4 hours. The obtained reactant (B1) (2.24 g) was used in the next step without being purified.

Next, compound (B1) (1.00 g, 7.7 mmol) and sodium hydroxide (620 mg, 15.4 mmol) were dissolved in a mixed solvent of tetrahydrofuran (THF) (39 mL) and water (39 mL). The obtained solution was reacted at room temperature for 20 hours. Next, iodine and potassium iodide were added to the reactant. The mixture was reacted at room temperature for 1 hour. The obtained reactant (C1) (727 mg) was used in the next step without being purified.

Next, SN-38 (220 mg, 0.56 mmol), a compound (C1) (83 mg, 0.28 mmol), 1-ethyl-3-(3-dimethylaminopropyl)carbodiimide hydrochloride (EDC·HCl) (214 mg, 1.12 mmol), and 4-dimethylaminopyridine (DMAP) (14 mg, 0.11 mmol) were dissolved in dichloromethane (DCM) (5.6 mL). The obtained solution was stirred at 40°C for 16 hours, and then the solvent was evaporated under reduced pressure to collect the residue. Next, the collected residue was purified by silica gel column chromatography (chloroform → chloroform/methanol = 50:1) to obtain a compound (1-1) (149 mg, yield: 51%, total yield: 32%) as a light yellow solid.

¹H NMR measurement results of compound (1-1) are shown below.

¹H NMR (CDCl₃, 400 MHz): δ = 7.66 (d, 2H, J = 2.5 Hz), 7.63 (d, 2H, J = 9.1 Hz), 7.30 (s, 2H), 7.23 (dd, 2H, J = 9.1 Hz, 2.5 Hz), 5.66 (d, 4H, J = 16.3 Hz), 5.26 to 5.19 (m, 4H), 5.09 (d, 4H, J = 18.7 Hz), 4.23 (s, 2H), 3.14 to 3.10 (m, 4H), 2.90 to 2.84 (m, 4H), 2.15 to 2.27 (m, 4H), 1.90 to 1.81 (m, 4H), 1.47 to 1.42 (m, 18H), 0.99 (t, 6H, J = 7.4Hz) ppm

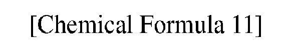

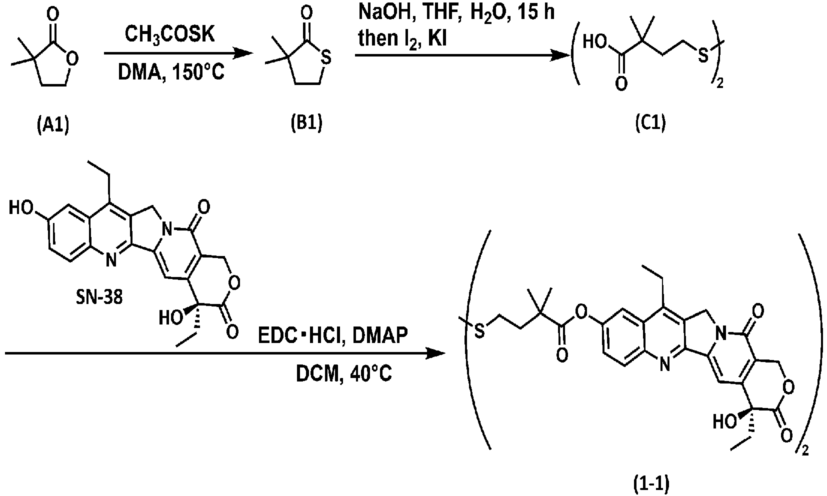

### [Production of nanoparticles]

### (Compound (1-1); Example 1)

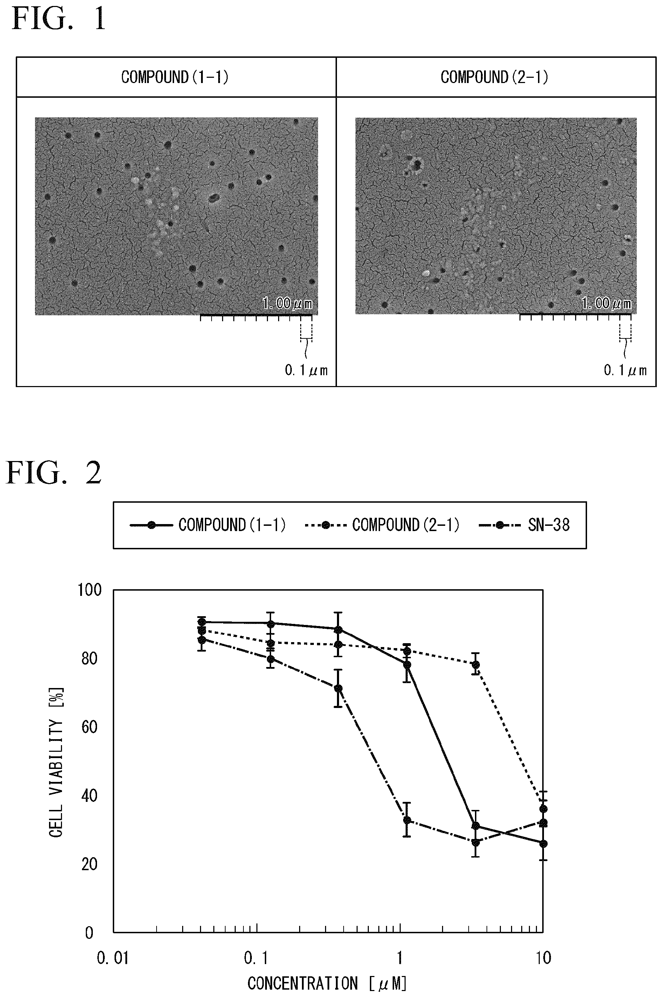

100 µL of a dimethyl sulfoxide solution of compound (1-1), prepared at 5 mM, was injected into 10 mL of water at room temperature using a syringe, and the mixture was stirred at 1,500 rpm for 2 seconds to obtain an aqueous dispersion liquid of nanoparticles. The final concentration of compound (1-1) in the aqueous dispersion liquid of the nanoparticles was 0.05 mM. It was confirmed by SEM and DLS that a particle diameter thereof was approximately 100 nm. FIG. 1 shows an SEM image of the nanoparticles of compound (1-1). A ClogP of compound (1-1) was 5.92.

### (Compound (2-1); Comparative Example 1)

An aqueous dispersion liquid of nanoparticles was obtained in the same manner as in Example 1, except that the following compound (2-1) was used instead of compound (1-1). The final concentration of compound (2-1) in the aqueous dispersion liquid of the nanoparticles was 0.05 mM. FIG. 1 shows an SEM image of the nanoparticles of compound (2-1).

Compound (2-1) was synthesized in the same manner as in Synthesis Example 1 described above, except that compound (L1) was used instead of compound (C1). A ClogP of compound (2-1) was 6.56.

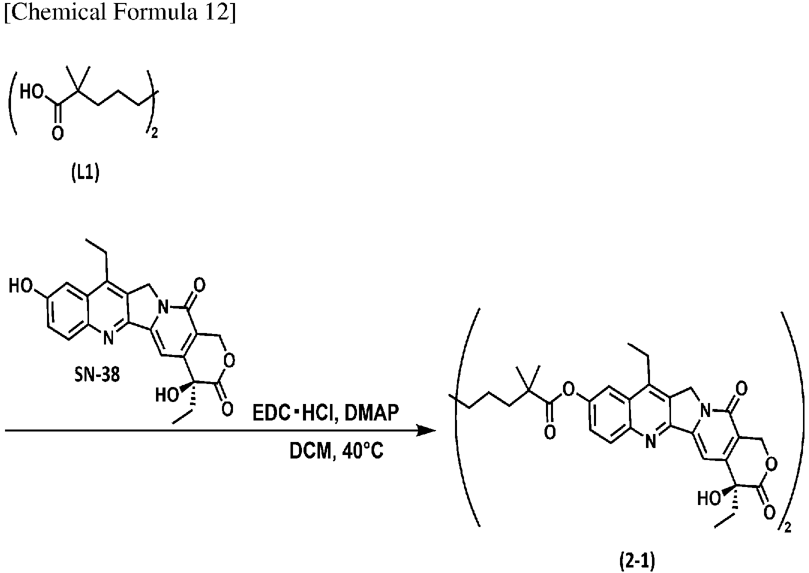

### (Compound (2-2); Comparative Example 2)

An aqueous dispersion liquid of nanoparticles was obtained in the same manner as in Example 1 described above, except that the following compound (2-2) was used instead of compound (1-1). The final concentration of compound (2-2) in the aqueous dispersion liquid of the nanoparticles was 0.05 mM.

Compound (2-2) was synthesized in the same manner (see below) as in Synthesis Example 1 described above, except that a compound (L2) was used instead of compound (C1). A ClogP of compound (2-2) was 4.50.

Specifically, compound (2-2) was synthesized by the following method.

SN-38 (580 mg, 1.48 mmol), 4,4-dithiodibutyric acid (176 mg, 0.74 mmol), 1-ethyl-3-(3-dimethylaminopropyl)carbodiimide hydrochloride (EDC·HCl) (564 mg, 2.96 mmol), and 4-dimethylaminopyridine (DMAP) (72 mg, 0.59 mmol) were dissolved in dichloromethane (DCM) (30 mL). The obtained solution was stirred at room temperature for 19 hours, and then the solvent was evaporated under reduced pressure to collect the residue. Next, the collected residue was purified by silica gel column chromatography (chloroform → chloroform/methanol = 50:1), and further purified by silica gel column chromatography (ethyl acetate) to obtain a compound (2-2) (97 mg, yield: 13%) as a light yellow solid.

1H NMR measurement results of compound (2-2) are shown below.

1H NMR (CDCl3, 400 MHz): δ = 7.73 (d, 2H, J = 9.1 Hz), 7.64 (d, 2H, J = 2.4Hz), 7.36 (s, 2H), 7.31 (dd, 2H, J = 9.1 Hz, 2.5 Hz), 5.65 (d, 2H, J = 16.3 Hz), 5.26 to 5.07 (m, 6H), 4.33 (s, 2H), 3.09 (t, 4H, J = 7.7 Hz), 2.96 to 2.90 (m, 4H), 2.89 to 2.84 (m, 4H), 2.29 to 2.22 (m, 4H), 1.92 to 1.78 (m, 4H), 1.43 (t, 6H, J = 7.7 Hz), 0.98 (t, 6H, J = 7.4Hz) ppm

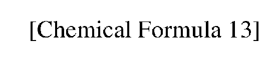

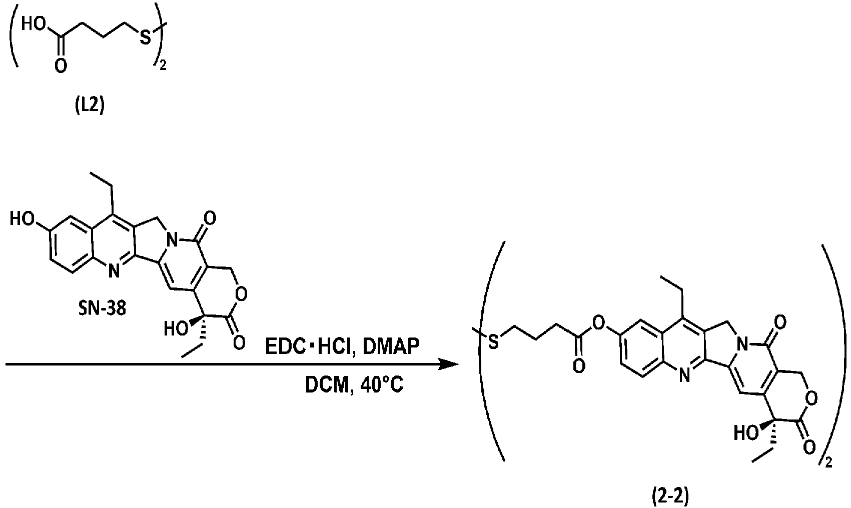

### (Compound (2-3); Comparative Example 3)

An aqueous dispersion liquid of nanoparticles was obtained in the same manner as in Example 1 described above, except that the following compound (2-3) was used instead of compound (1-1). The final concentration of compound (2-3) in the aqueous dispersion liquid of the nanoparticles was 0.05 mM.

Compound (2-3) was synthesized in the same manner (see below) as in Synthesis Example 1 described above, except that a compound (L3) was used instead of compound (C1). A ClogP of compound (2-3) was 5.14.

Specifically, compound (2-3) was synthesized by the following method.

SN-38 (100 mg, 0.26 mmol), sebacic acid (26 mg, 0.13 mmol), 1-ethyl-3-(3-dimethylaminopropyl)carbodiimide hydrochloride (EDC·HCl) (97 mg, 0.51 mmol), and 4-dimethylaminopyridine (DMAP) (6 mg, 0.05 mmol) were dissolved in dichloromethane (DCM) (5.1 mL). The obtained solution was stirred at room temperature for 19 hours, and then the solvent was evaporated under reduced pressure to collect the residue. Next, the collected residue was purified by silica gel column chromatography (chloroform → chloroform/methanol = 50:1) to obtain a compound (2-3) (109 mg, yield: 90%) as a light yellow solid.

1H NMR measurement results of compound (2-3) are shown below.

1H NMR (CDCl3, 400 MHz): δ = 7.97 (d, 2H, J = 9.1 Hz), 7.77 (d, 2H, J = 2.5 Hz), 7.49 (s, 2H), 7.43 (dd, 2H, J = 9.1 Hz, 2.5 Hz), 5.72 (d, 2H, J = 16.3 Hz), 5.29 to 5.16 (m, 6H), 3.92 (s, 2H), 3.15 (q, 4H, J = 7.8 Hz), 2.68 (t, 4H, J = 7.2 Hz), 1.93 to 1.82 (m, 8H), 1.53 to 1.49 (m, 8H), 1.43 (t, 6H, J = 7.7 Hz), 1.02 (t, 6H, J = 7.4 Hz) ppm

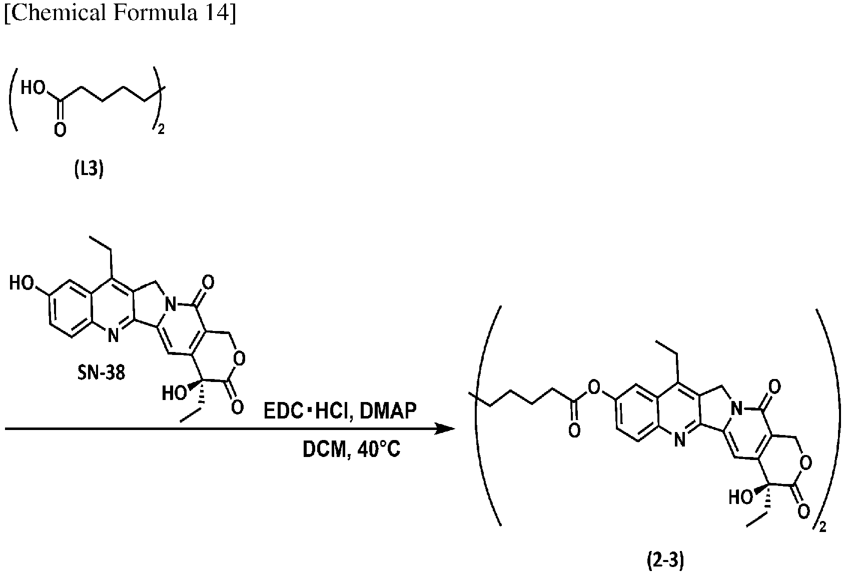

### (SN-38; Comparative Example 4)

An aqueous dispersion liquid of nanoparticles was obtained in the same manner as in Example 1, except that SN-38 was used instead of compound (1-1). The final concentration of SN-38 in the aqueous dispersion liquid of the nanoparticles was 0.1 mM.

### [In vitro activity test]

A human breast cancer cell line MCF-7 was suspended in Dulbecco's modified Eagle's medium (DMEM) to which 10% fetal bovine serum (FBS) was added, thereby preparing a cell suspension of 2 × 10⁵ cells/mL. The above-described cell suspension was seeded on a 96-well plate at 100 µL per well. The next day, the aqueous dispersion liquid of the nanoparticles of compound (1-1), the aqueous dispersion liquid of the nanoparticles of compound (2-1), or the aqueous dispersion liquid of the nanoparticles of SN-38, prepared as described above, was added to the MCF-7 cell culture solution so that the concentration thereof was 0.04 to 10 µM (in terms of SN-38). Thereafter, the cells were cultured at 37°C and 5% CO₂ for 48 hours. Thereafter, cell viability was measured by a colorimetric method using Cell Counting Kit-8 (manufactured by DOJINDO LABORATORIES.) and a microplate reader.

The results are shown in FIG. 2. Compounds (1-1) and (2-1) had a higher cell viability of MCF-7 compared with SN-38. This result is considered to be due to the fact that cytotoxic activity against MCF-7 was decreased by dimerization of compound (1-1) and compound (2-1). It is considered that compound (1-1) and compound (2-1) exhibited antitumor activity by cleaving the linker moiety to form a monomer. In compound (1-1), the cell viability of MCF-7 was lower than that of compound (2-1). This result is considered to be due to the fact that the linker moiety of compound (1-1) was more likely to be cleaved in the MCF-7 cell compared with compound (2-1). From the above-described results, it is considered that compound (1-1) could effectively exhibit an anticancer action by reducing and cleaving the disulfide bond of the linker moiety by glutathione in cancer cells.

### [Drug release test in PBS under reduced dithiothreitol treatment (1)]

Dithiothreitol (DTT) is a low-molecular-weight reducing agent. DTT has a linear structure (Formula (1)) in a reduced state, and a 6-membered ring (Formula (2)) by a disulfide bond in an oxidized state. DTT reduces a disulfide bond of a protein to cysteine. For this reason, DTT may be used to prevent the formation of an intramolecular or intermolecular disulfide bond between cysteine residues of a protein.

In the present test, the release of SN-38 from compound (1-1) and compound (2-1) in PBS to which reduced DTT was added was evaluated.

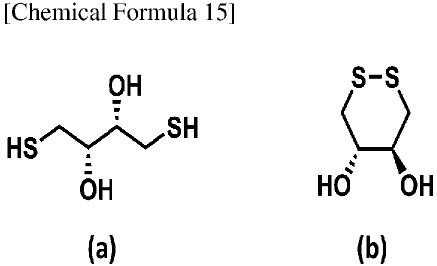

A solution was prepared by adding an aqueous dispersion liquid (5 µM, 500 µL) of nanoparticles of compound (1-1) or an aqueous dispersion liquid (5 µM, 500 µL) of nanoparticles of compound (2-1) to 500 µL of PBS (-) to which 10 mM of reduced dithiothreitol (DTT) had been added. "PBS (-)" used hereinafter is phosphate-buffered saline (pH: 7.4) containing NaCl (137 mM), KCl (2.7 mM), Na₂HPO₄ (10 mM), and KH₂PO₄ (1.76 mM).

The above-described reaction solution was incubated at 37°C for 1 hour or 24 hours. 200 µL of the reaction solution after incubation was collected. The collected reaction solution was mixed with 800 µL of a 3% acetic acid solution of acetonitrile/methanol (1/1). The obtained mixed solution was analyzed by high-performance liquid chromatography (detection wavelength: 365 nm) as an analysis target sample.

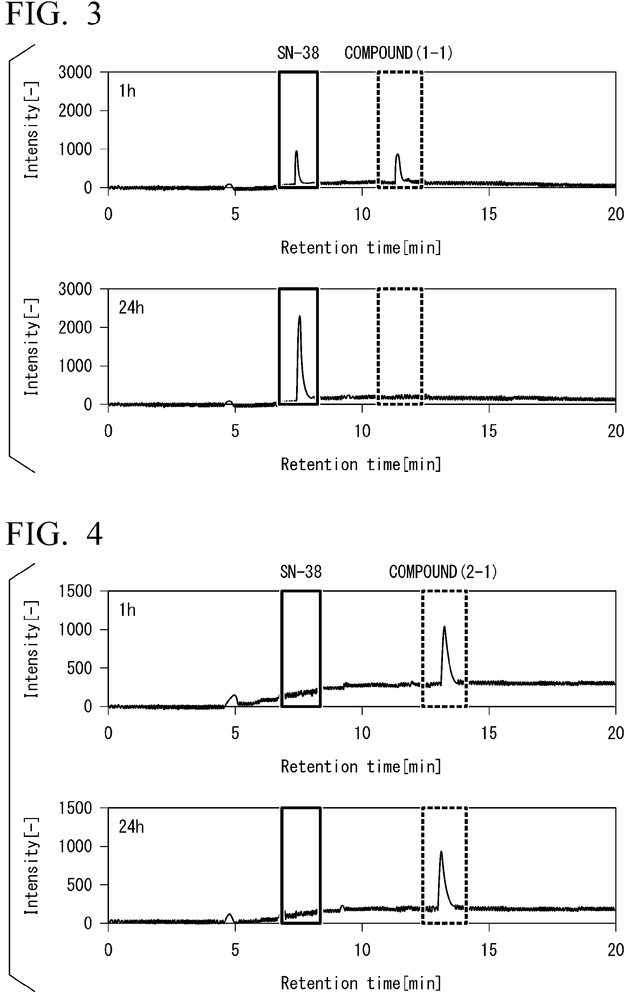

The results are shown in FIGS. 3 and 4. FIG. 3 shows the results of compound (1-1), and FIG. 4 shows the results of compound (2-1). In compound (1-1), it was confirmed that the disulfide bond was cleaved by DTT treatment, and SN-38 was released. On the other hand, in compound (2-1), the release of SN-38 was not observed.

Furthermore, for compounds (2-2) and (2-3), a drug release test in PBS by the reduced dithiothreitol treatment was also carried out according to the same method as described above. As a result, it was confirmed that the results were the same as those in the drug release test in PBS by the reduced glutathione treatment, including the above-described results of compounds (1-1) and (2-1). That is, in compound (2-2), the amounts of compound (2-2) and SN-38 were substantially equal to each other with the passage of time, and compound (2-2) disappeared with the further passage of time. From the above, it was found that compound (2-2) was very easily decomposed. In addition, in compound (2-3), compound (2-3) was decreased with the passage of time and SN-38 was increased with the passage of time, but the rate of increase of SN-38 was slower than that of compound (1-1).

### [Drug release test in PBS without reduced dithiothreitol treatment (1)]

A solution was prepared by adding the aqueous dispersion liquid (5 µM, 500 µL) of the nanoparticles of compound (1-1) or the aqueous dispersion liquid (5 µM, 500 µL) of the nanoparticles of compound (2-1) to 500 µL of PBS (-).

The above-described reaction solution was incubated at 37°C for 1 hour or 24 hours. 200 µL of the reaction solution was collected after the incubation. The collected reaction solution was mixed with 800 µL of a 3% acetic acid solution of acetonitrile/methanol (1/1). The obtained mixed solution was analyzed by high-performance liquid chromatography (detection wavelength: 365 nm) as an analysis target sample.

The results are shown in FIGS. 5 and 6. FIG. 5 shows the results of compound (1-1), and FIG. 6 shows the results of compound (2-1).

In both compound (1-1) and compound (2-1), it was confirmed that the compound was stable in PBS without dithiothreitol treatment.

Furthermore, for compounds (2-2) and (2-3), a drug release test in PBS without the dithiothreitol treatment was also carried out according to the same method as described above. As a result, in both compound (2-2) and compound (2-3), it was confirmed that the compound was stable in PBS not treated by dithiothreitol.

### [Drug release test in PBS under reduced dithiothreitol treatment and without reduced dithiothreitol treatment (2)]

A drug release test was carried out according to the same method as described above, except that, in the "Drug release test in PBS under reduced dithiothreitol treatment and without reduced dithiothreitol treatment (1)" described above, the test concentration of the nanoparticles in the aqueous dispersion liquid of the nanoparticles of each of compound (1-1) and compounds (2-1) to (2-3) was changed from "5 µM" to "50 µM", and "PBS (-)" in the reaction solution was changed to "PBS/EtOH (7/3) (pH: 7.4)". As a result, it was confirmed that the same results as those in "Drug release test in PBS under reduced glutathione treatment and without reduced glutathione treatment" described later were obtained with any of compound (1-1) and compounds (2-1) to (2-3). PBS/EtOH (7/3) (pH: 7.4) is a solvent obtained by mixing PBS (-) and ethanol at a volume ratio of 7:3.

### [Drug release test in PBS under reduced glutathione treatment]

Glutathione (GSH) is a tripeptide consisting of three amino acids (glutamic acid, cysteine, and glycine). GSH has an amide bond between an amino group of cysteine and a carboxyl group on the side chain side of glutamic acid, such an amide bond is not usually observed. Reduced GSH (Formula (c)) acts as an electron donor and reduces a disulfide bond of the protein to cysteine. For this reason, the reduced GSH may be used to prevent formation of an intramolecular or intermolecular disulfide bond between cysteine residues of a protein.

In the present test, the release of SN-38 from compound (1-1) and compounds (2-1) to (2-3) in PBS to which the reduced GSH was added was evaluated.

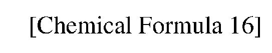

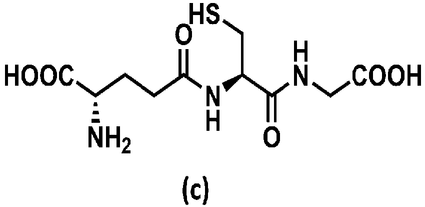

A reaction solution was prepared by adding the aqueous dispersion liquid (50 µM, 500 µL) of the nanoparticles of compound (1-1) or the aqueous dispersion liquid (50 µM, 500 µL) of the nanoparticles of any one of compounds (2-1) to (2-3) to 500 µL of PBS (-)/EtOH (7/3) (pH: 7.4) to which 10 mM of reduced glutathione (GSH) had been added.

The above-described reaction solution was incubated at 37°C for 1 hour, 6 hours, 24 hours, or 48 hours. 200 µL of the reaction solution was collected after the incubation. The collected reaction solution was mixed with 800 µL of a 3% acetic acid solution of acetonitrile/methanol (1/1). The obtained mixed solution was analyzed by high-performance liquid chromatography (detection wavelength: 365 nm) as an analysis target sample.

The results are shown in FIGS. 7 to 10. FIGS. 7 to 10 show the results of compound (1-1) and compounds (2-1) to (2-3), respectively. In compound (1-1), compound (1-1) was reduced and SN-38 was increased with the passage of time (FIG. 7). This is considered to be because the disulfide bond was cleaved by GSH to release SN-38.

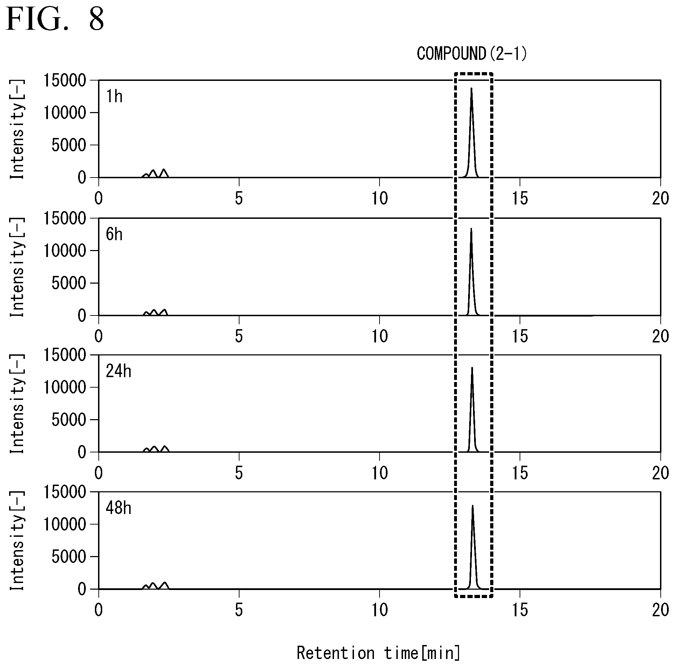

On the other hand, in compound (2-1), the release of the compound SN-38 was not confirmed even after 48 hours (FIG. 8). This result indicates that compound (2-1) was not decomposed by GSH.

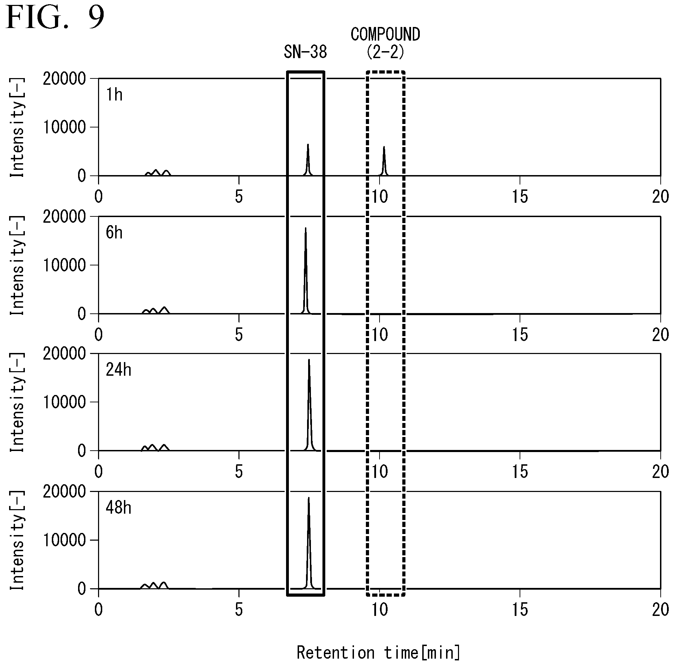

In compound (2-2), after 1 hour, the amounts of compound (2-2) and SN-38 were almost the same, and compound (2-2) disappeared after 6 hours (FIG. 9). This result indicates that compound (2-2) was very easily decomposed.

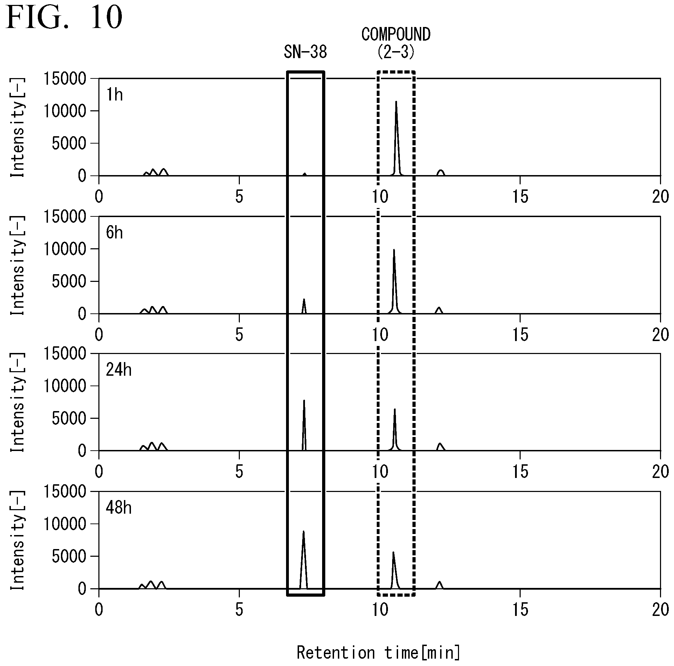

In compound (2-3), compound (2-3) was reduced and SN-38 was increased with the passage of time (FIG. 10). However, the rate of increase in SN-38 was slower than that of compound (1-1).

### [Drug release test in PBS without reduced glutathione treatment]

A reaction solution was prepared by adding each of the aqueous dispersion liquid (50 µM, 500 µL) of the nanoparticles of compounds (1-1) and (2-1) to (2-3) to 500 µL of PBS (-)/EtOH (7/3) (pH: 7.4).

The above-described reaction solution was incubated at 37°C for 1 hour, 6 hours, or 24 hours. 200 µL of the reaction solution was collected after the incubation. The collected reaction solution was mixed with 800 µL of a 3% acetic acid solution of acetonitrile/methanol (1/1). The obtained mixed solution was analyzed by high-performance liquid chromatography (detection wavelength: 365 nm) as an analysis target sample.

In any of compounds (1-1) and (2-1) to (2-3), it was confirmed that the compound was stable in PBS without GSH treatment.

From the above-described results, it was presumed that the nanoparticles containing compound (1-1) did not release SN-38 in a healthy tissue having a low GSH concentration, and continuously released SN-38 in a cancer tissue having a high GSH concentration. Therefore, it was considered that compound (1-1) could be suitably used for treatment of cancer. On the other hand, it was presumed that the nanoparticles containing compounds (2-1) and (2-3) did not or did not sufficiently release SN-38 even in the cancer tissue. It was presumed that the nanoparticles containing compound (2-2) released SN-38 in the cancer tissue having a high GSH concentration, but since SN-38 was released in a short time, it was presumed that the nanoparticles had poor sustainability.

### [Synthesis Example 2]

Allyl bromide (25.6 mL, 25.6 mmol) was added dropwise to a tetrahydrofuran (THF) solution (12 mL) of lithium bis(trimethylsilyl)amide (LHMDS) (25.6 mL, 25.6 mmol) at -78°C, reacted at -25°C for 0.5 hours, and the reaction solution was cooled again to -78°C. Subsequently, γ-butyrolactone (1.0 g, 11.6 mmol) was added dropwise thereto, and the mixture was gradually heated to room temperature (RT) and reacted at the same temperature overnight. A saturated aqueous ammonium chloride solution was added to stop the reaction, and the mixture was extracted with ethyl acetate. The organic layer was washed with saturated saline, and dried over anhydrous sodium sulfate. A crude product obtained by distilling off the organic solvent under reduced pressure was purified by silica gel column chromatography to obtain a compound (A2) (1.53 g, yield: 79%) as a light brown oily substance. Each spectrum of compound (A2) matched the reference literature (Organic Letters (2015), 17 (14), pp. 3604 to 3607).

Next, compound (A2) (300 mg, 1.80 mmol) and Hoveyda-Grubbs second generation catalyst (HG-II) (15.0 mg, 0.0177 mmol) were dissolved in dichloromethane (DCM) (7.50 mL). The obtained solution was reacted at room temperature for 1 hour. A crude product obtained by distilling off the solvent of the reactant under reduced pressure was purified by silica gel column chromatography to obtain a compound (B2) (212 mg, yield: 85%) as a colorless oily substance.

Next, the obtained compound (B2) (221 mg, 1.53 mmol) was dissolved in methanol (5.0 mL), and hydrogen and palladium carbon (Pd/C) (21 mg) were added to the methanol solution at room temperature. The mixture was reacted at the same temperature for 3 hours in a hydrogen gas atmosphere. After completion of the reaction, a filtrate obtained by celite filtration was distilled off under reduced pressure to obtain a crude product (220 mg) of a compound (C2).

Next, potassium thioacetate (CH₃COSK) (896 mg, 7.85 mmol) was added to a solution of compound (C2) (220 mg, 1.57 mmol) in dimethylacetamide (DMA) (1.1 mL) at room temperature, and the mixture was reacted at 150°C for 5 hours. Water was added to stop the reaction, and a target substance was extracted with ethyl acetate. The organic layer was washed with water, and dried over anhydrous sodium sulfate. A crude product obtained by distilling off the organic solvent from the dried organic layer under reduced pressure was purified by silica gel column chromatography to obtain a compound (D2) (235 mg, yield: 96%) as a dark brown oily substance.

Next, potassium hydroxide (139 mg, 2.48 mmol) was added to an aqueous solution (5.6 mL) of the obtained compound (D2) (176 mg, 1.12 mmol) at room temperature, and the mixture was reacted at 110°C for 0.5 hours. The obtained reaction solution was cooled to room temperature, iodine (85.3 mg, 0.366 mmol) and potassium iodide (55.8 mg, 0.366 mmol) were added thereto, and the mixture was further stirred at room temperature for 1.5 hours. Water and anhydrous sodium thiosulfate were added to stop the reaction. 1 M hydrochloric acid aqueous solution was added to the reactant, and a target substance was extracted with ethyl acetate. The organic layer was washed with water, and dried over anhydrous sodium sulfate. The organic solvent was distilled off from the dired organic layer under reduced pressure to obtain a crude product (180 mg) of a compound (E2) as a black gum-like substance.

Next, SN-38 (158 mg, 0.403 mmol), compound (E2) (55.9 mg, 0.161 mmol), 1-ethyl-3-(3-dimethylaminopropyl)carbodiimide hydrochloride (EDC·HCl) (154 mg, 0.805 mmol), and 4-dimethylaminopyridine (DMAP) (1.9 mg, 0.0161 mmol) were dissolved in dichloromethane (DCM) (2.7 mL), and the obtained solution was reacted at 40°C for 2 days. A saturated aqueous ammonium chloride solution was added to stop the reaction, and a target substance was extracted with chloroform. The organic layer was washed with saturated saline, and dried over anhydrous magnesium sulfate. A crude product obtained by distilling off the organic solvent from the dried organic layer under reduced pressure was purified by silica gel column chromatography (chloroform → chloroform/methanol = 80:1) to obtain a compound (1-5) (41.2 mg, total yield: 23%) as a light yellow solid.

The analysis result of compound (1-5) by HR-MS (ESI-TOF) is shown below. m/z C₆₀H₆₃N₄O¹²S₂⁺ ([M+N]⁺) calcd for 1095.3878; found 1095.3871

¹H NMR measurement results of compound (1-5) are shown below.

¹H NMR (DMSO-d6, 400 MHz): δ = 0.87 (t, J = 7.4 Hz, 6H), 1.27 (t, J = 7.6 Hz, 6H), 1.69 (brs, 12H), 1.82 to 1.90 (m, 4H), 2.18 to 2.26 (m, 8H), 2.85 to 2.89 (m, 4H), 3.08 to 3.13 (m, 4H), 5.16 (s, 4H), 5.40 (s, 4H), 6.51 (s, 2H), 7.21 (s, 2H), 7.50 (dd, J = 9.1 Hz, 2.5 Hz, 2H), 7.87 (d, J = 2.4 Hz, 2H), 7.99 (d, J = 9.1 Hz, 2H) ppm

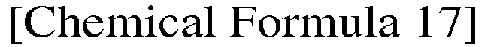

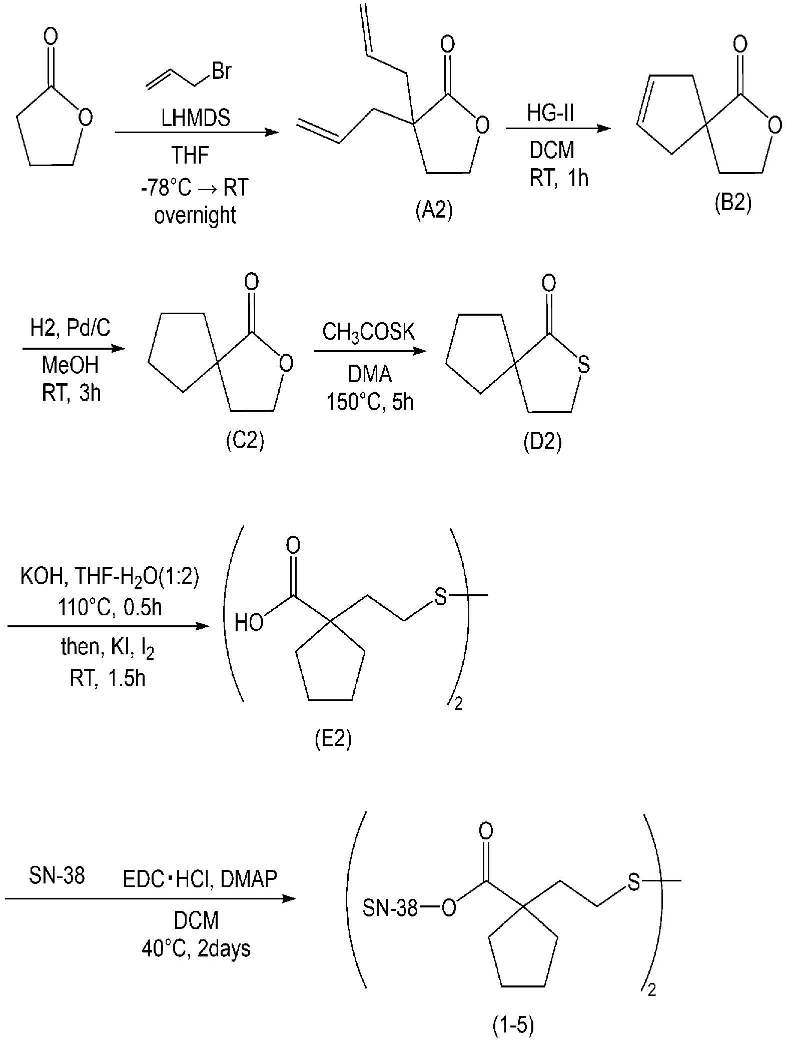

### [Synthesis Example 3]

δ-Valerolactone (455 mg, 4.55 mmol) and iodomethane (1.42 g, 10 mmol) were dissolved in tetrahydrofuran (THF) (8 mL). Lithium bis(trimethylsilyl)amide (LiHMDS) (1.0 M THF solution) (10 mL) was added to the obtained solution at -78°C. The obtained solution was reacted at -78°C for 40 minutes, heated from -78°C to -40°C, and further reacted at -40°C for 4.5 hours. The obtained reactant was purified by silica gel chromatography to obtain a compound (A3) (330 mg, yield: 57%) as a yellow oil.

Next, compound (A3) (285 mg, 2.23 mol) and potassium thioacetate (CH₃COSK) (407 mg, 3.56 mmol) were dissolved in dimethylacetamide (DMA) (5 mL). The obtained solution was reacted at 150°C for 2.5 hours. The obtained reactant was purified by silica gel chromatography to obtain a compound (B3) (190 mg, yield: 60%) as a yellow oil.

Next, compound (B3) (184 mg, 1.28 mmol) and sodium hydroxide (102 mg, 2.55 mmol) were dissolved in a mixed solvent of tetrahydrofuran (THF) and water (1:1; 16 mL). The obtained solution was reacted at room temperature for 25 hours. Next, iodine and potassium iodide were added to the reactant, and the mixture was reacted at room temperature for 1.25 hours. The obtained reactant (C3) was used in the next step without being purified.

Next, SN-38 (235 mg, 0.6 mmol), compound (C3) (97 mg, 0.3 mmol), 1-ethyl-3-(3-dimethylaminopropyl)carbodiimide hydrochloride (EDC·HCl) (230 mg, 1.2 mmol), and 4-dimethylaminopyridine (DMAP) (146 mg, 1.2 mmol) were dissolved in dichloromethane (DCM) (6 mL). The obtained solution was reacted at 40°C overnight, and then the solvent was evaporated under reduced pressure to collect the residue. Next, the collected residue was purified by silica gel column chromatography (chloroform → chloroform/methanol = 29:1) to obtain a compound (1-6) (230 mg, yield: 72%) as a light yellow solid.

¹H NMR measurement results of compound (1-6) are shown below.

¹H NMR (CDCl₃, 400 MHz): δ = 1.00 (t, J = 7.4 Hz, 6H), 1.37 to 1.42 (m, 18H), 1.83 to 1.92 (m, 12H), 2.78 to 2.80 (m, 4H), 3.04 to 3.10 (m, 4H), 4.09 (s, 2H), 5.10 to 5.28 (m, 6H), 5.71 (d, J = 16.3 Hz, 2H), 7.41 (dd, J = 9.1 Hz, 2.5 Hz, 2H), 7.48 (s, 2H), 7.69 (d, J = 9.1 Hz, 2H), 7.97 (d, J = 2.5 Hz, 2H)

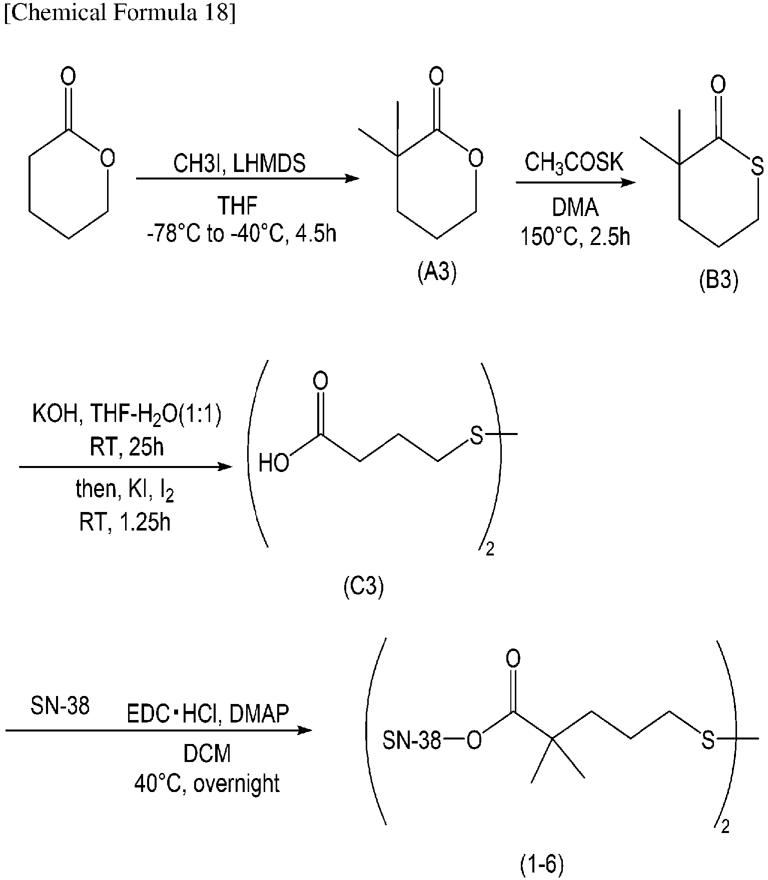

### [Synthesis Example 4]

Allyl bromide (25.6 mL, 25.6 mmol) was added dropwise to a tetrahydrofuran (THF) solution (12 mL) of lithium bis(trimethylsilyl)amide (LHMDS) (25.6 mL, 25.6 mmol) at -78°C, reacted at -25°C for 0.5 hours, and the reaction solution was cooled again to -78°C. Subsequently, γ-butyrolactone (1.0 g, 11.6 mmol) was added dropwise thereto, and the mixture was gradually heated to room temperature (RT) and reacted at the same temperature overnight. A saturated aqueous ammonium chloride solution was added to stop the reaction, and the mixture was extracted with ethyl acetate. The organic layer was washed with saturated saline, and dried over anhydrous sodium sulfate. A crude product obtained by distilling off the organic solvent under reduced pressure was purified by silica gel column chromatography to obtain a compound (A2) (1.53 g, yield: 79%) as a light brown oily substance. Each spectrum of compound (A2) matched the reference literature (Organic Letters (2015), 17 (14), pp. 3604 to 3607).

Next, compound (A2) (150 mg, 0.902 mmol) was dissolved in methanol (4.5 mL), and hydrogen and palladium carbon (Pd/C) (15 mg) were added to the methanol solution at room temperature. The mixture was reacted at the same temperature for 3 hours in a hydrogen gas atmosphere. After completion of the reaction, a filtrate obtained by celite filtration was distilled off under reduced pressure to obtain a crude product of a compound (B4).

Next, potassium thioacetate (CH₃COSK) (237 mg, 2.07 mmol) was added to a solution of the crude product (0.902 mmol) of compound (B4) in dimethylacetamide (DMA) (0.9 mL) at room temperature, and then the mixture was reacted at 150°C for 5 hours. Water was added to stop the reaction, and a target substance was extracted with ethyl acetate. The organic layer was washed with water, and dried over anhydrous sodium sulfate. A crude product obtained by distilling off the organic solvent from the dried organic layer under reduced pressure was purified by silica gel column chromatography to obtain a compound (C4) (67.9 mg, yield: 40%) as a light yellow oily substance.

Next, an aqueous solution (4.8 mL) of potassium hydroxide (45.0 mg, 0.803 mmol) was added to the THF aqueous solution (2.4 mL) of the obtained compound (C4) (67.9 mg, 0.364 mmol) at room temperature, and the mixture was reacted at 110°C for 1 hour. The obtained reaction solution was cooled to room temperature, iodine (25.3 mg, 0.109 mmol) and potassium iodide (12.0 mg, 0.728 mmol) were added thereto, and the mixture was further stirred at room temperature for 1.5 hours. Water and anhydrous sodium thiosulfate were added to stop the reaction. 1 M hydrochloric acid aqueous solution was added to the reactant, and a target substance was extracted with ethyl acetate after the solution was acidified. The organic layer was washed with water, and dried over anhydrous sodium sulfate. The organic solvent was distilled off from the dried organic layer under reduced pressure to obtain a crude product (63.6 mg) of a compound (D4) as a yellow gum-like substance.

Next, SN-38 (135 mg, 0.334 mmol), compound (D4) (63.6 mg, 0.157 mmol), 1-ethyl-3-(3-dimethylaminopropyl)carbodiimide hydrochloride (EDC·HCl) (75.2 mg, 0.397 mmol), and 4-dimethylaminopyridine (DMAP) (3.80 mg, 0.0314 mmol) were dissolved in dichloroethane (DCE) (3.4 mL), and then the obtained solution was reacted at 80°C for 24 hours. The solvent was distilled off under reduced pressure to collect the residue. Next, the collected residue was purified by silica gel column chromatography (chloroform → chloroform/methanol = 50:1) to obtain a compound (1-3).

The obtained compound (1-3) was confirmed by ¹H NMR measurement (DMSO-d6, 400 MHz).

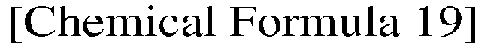

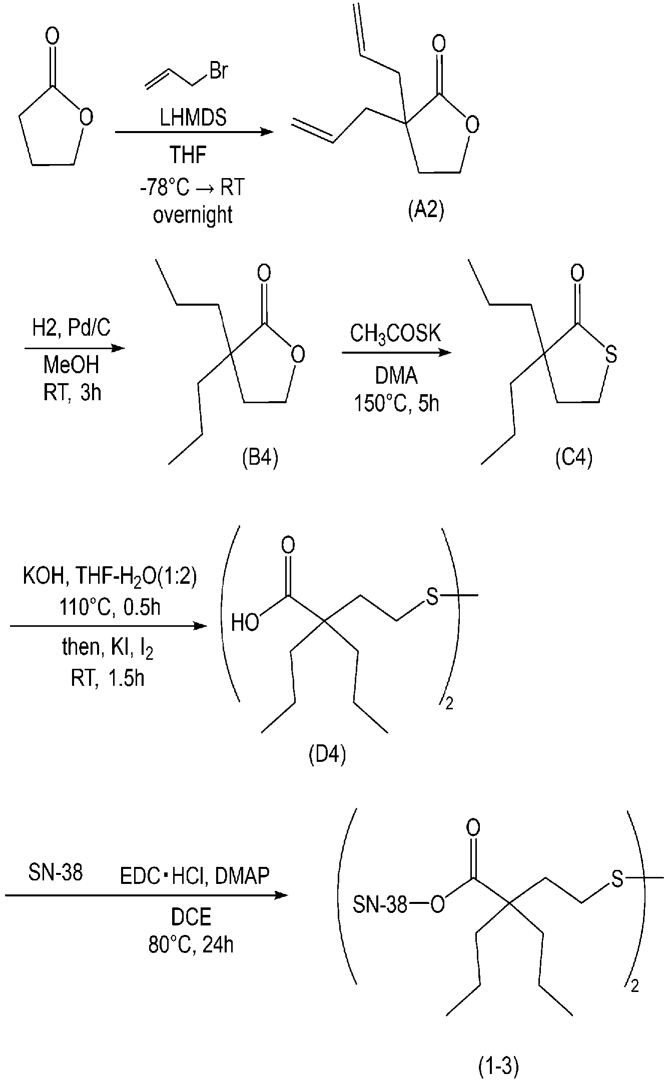

Although preferred examples of the present invention have been described above, the present invention is not limited to these examples. It is possible to add other configurations or to omit, replace, or modify the configurations described herein without departing from the spirit of the present invention. The present invention is not limited by the above description, but only by the scope of the appended claims.

## Claims

1. A compound represented by General Formula (1) or a salt thereof, 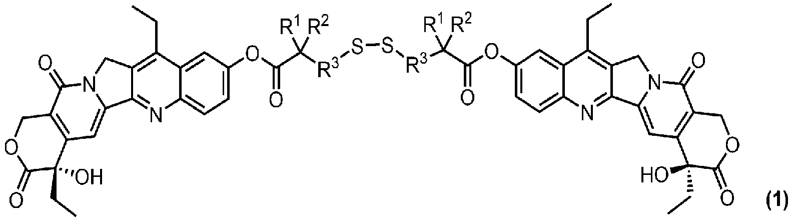 [in the formula, each of R¹ and R² independently represents an alkyl group having 1 to 4 carbon atoms, R¹ and R² may be bonded to each other to form a ring together with a carbon atom to which R¹ and R² are bonded, and each R³ independently represents an alkylene group having 1 to 4 carbon atoms].

2. The compound or a salt thereof according to Claim 1,
wherein ClogP is 5.0 to 14.0.

3. The compound or a salt thereof according to Claim 1 or 2,
wherein R¹ and R² in General Formula (1) are methyl groups.

4. The compound or a salt thereof according to Claim 1 or 2,
wherein R¹ and R² in General Formula (1) are ethyl groups.

5. The compound or a salt thereof according to Claim 1 or 2,
wherein R³ in General Formula (1) is an ethylene group.

6. The compound or a salt thereof according to Claim 1 or 2,
wherein R³ in General Formula (1) is a propylene group.

7. Nanoparticles comprising:
the compound or a salt thereof according to Claim 1 or 2.

8. A medicine comprising:
the compound or a salt thereof according to Claim 1 or 2.

9. A medicine comprising:
the nanoparticle according to Claim 7.

10. The medicine according to Claim 8,
wherein the medicine is used for prevention or treatment of cancer.

11. The medicine according to Claim 9,
wherein the medicine is used for prevention or treatment of cancer.

12. A method for producing nanoparticles, comprising:
a step of injecting a water-miscible organic solvent solution of the compound or a salt thereof according to Claim 1 or 2 into water.
